# FASCICULE DE BREVET EUROPEEN

(11) **EP 3 223 774 B1**
(45) Date de publication et mention de la délivrance du brevet: **30.09.2020**
(21) Numéro de dépôt: 15794172.5
(22) Date de dépôt: 12.11.2015
(51) Int. Cl.: A61K 8/25, A61K 8/73, A61K 8/02, A61Q 19/00

(54) **PANSEMENT OU PATCH ADHÉSIF À FORTE CHARGE MINÉRALE**
ADHESIVER VERBAND ODER PATCH MIT HOHEM MINERALISCHEM FÜLLERGEHALT
ADHESIVE PLASTER OR PATCH WITH HIGH MINERAL CHARGE

(30) Priorité: 27.11.2014 FR 1461592
(43) Date de publication de la demande: 04.10.2017
(73) Titulaire: Silab S.A, 19130 Objat (FR)
(72) Inventeur: CABARET, Cyrille, F-19230 Segur le Chateau (FR); BENETOLLO, Camille, F-19100 Brive (FR); GEORGES, Elodie, F-87570 Rilhac-Rancon (FR); CLAUS, AGNES, JEANNE, MARIE-LOUISE, 87100 Limoges (FR); GLOAGUEN, VINCENT, MAURICE, 87700 Aixe sur Vienne (FR)
(74) Mandataire: Aquinov
(86) Numéro de dépôt international: PCT/EP2015/076419
(87) Numéro de publication internationale: WO 2016/083141

(56) Documents cités:
- WO-A1-2014/027873
- US-A1- 2006 034 905
- MARIA D. SANCHEZ-GARCIA ET AL: "Nanobiocomposites of Carrageenan, Zein, and Mica of Interest in Food Packaging and Coating Applications", JOURNAL OF AGRICULTURAL AND FOOD CHEMISTRY, vol. 58, no. 11, 9 juin 2010 (2010-06-09), pages 6884-6894, XP055203392, ISSN: 0021-8561, DOI: 10.1021/jf1007659
- SIBEL TUNÇ ET AL: "Preparation and characterization of biodegradable methyl cellulose/montmorillonite nanocomposite films", APPLIED CLAY SCIENCE, vol. 48, no. 3, 1 avril 2010 (2010-04-01), pages 414-424, XP055203320, ISSN: 0169-1317, DOI: 10.1016/j.clay.2010.01.016
- WILHELM H-M ET AL: "Starch films reinforced with mineral clay", CARBOHYDRATE POLYMERS, APPLIED SCIENCE PUBLISHERS, LTD. BARKING, GB, vol. 52, no. 2, 1 mai 2003 (2003-05-01), pages 101-110, XP004405537, ISSN: 0144-8617, DOI: 10.1016/S0144-8617(02)00239-4
- DE CARVALHO A J F ET AL: "A first insight on composites of thermoplastic starch and kaolin", CARBOHYDRATE POLYMERS, APPLIED SCIENCE PUBLISHERS, LTD. BARKING, GB, vol. 45, no. 2, 1 juin 2001 (2001-06-01), pages 189-194, XP004231831, ISSN: 0144-8617, DOI: 10.1016/S0144-8617(00)00315-5
- D. SARAVANAN ET AL: "Enhancement of thermal stability in the presence of crosslinking using natural biopolymer", ELIXIR APPL. CHEM, vol. 44, 1 janvier 2012 (2012-01-01), pages 7374-7377, XP055203389,

## Description

### DOMAINE DE L'INVENTION

La présente invention concerne un pansement adhésif ou patch adhésif et soluble comprenant un film soluble d'un polymère d'origine naturelle à forte teneur en minéraux. Elle concerne également ces pansements ou patchs pour leur utilisation en cosmétique ou en thérapie.

### ÉTAT DE LA TECHNIQUE

On connaît des pansements solubles sous forme de feuilles à base de polymères solubles dans l'eau, par exemple pour l'hydratation et le soin de la peau, notamment pour apporter des actifs sur la peau au point d'application du pansement, comme des huiles essentielles ou des extraits de plantes (EP 7 50 905, EP 613 692). De tels films solubles peuvent aussi être employés pour des « bains de bouche » en se dissolvant pour libérer des actifs une fois pris dans la bouche (US 7,491,406).

L'incorporation de charges minérales dans des compositions de polymères d'origine naturelle a fait l'objet de nombreuses études scientifiques sur les propriétés physicochimiques de tels mélanges. Sanchez Garcia & al. décrivent les propriétés barrières de compositions à base de carraghénanes auxquelles on ajoute différentes charges, dont de l'argile et de la zéine (J. Agric. Food Chem. 2010, 58, 6884-6894). Tunç & Duman décrivent la préparation et la caractérisation de films nanocomposites à base de méthyl cellulose et de montmorillonite (Applied Clay Science, 5010, 48, 414-424). Wilhelm & al. décrit l'étude des propriétés physicochimiques de films à base d'amidons, renforcé avec de l'argile (Carbohydrate Polymers, 2003, 52, 101-110). Carvalho & al. décrit l'analyse de compositions thermoplastiques à base d'amidon et de kaolin (Carbohydrate Polymers, 2001, 45, 189-194). Savanan & al. décrit l'amélioration de la stabilité thermique de compositions à base de chitine et de bentonite au moyen d'un agent de réticulation (Elixir Appl. Chem., 2012, 44, 7374-7377).

Toutefois, aucun de ces documents ne décrit de films susceptibles d'être employés comme pansement ou patch adhésif, en cosmétique ou en thérapie.

On notera au surplus que ce qui est identifié comme « film » dans ces publications, est généralement un produit rigide et cassant, qui s'apparente plus à des chips qui ne correspond pas à un film susceptible d'être employé comme pansement ou patch adhésif, en cosmétique ou en thérapie. C'est le cas en particulier du produit décrit par Tunç & Duman.

Il est d'ailleurs connu que dans de tels pansement ou patchs, les films à base de polyosides d'origine naturelle ne comprennent pas de fortes teneurs en charges minérales, tout au plus 15 % (US 2006/027873). Ou encore US 2006/0034905.

Il reste toutefois nécessaire d'améliorer ces films solubles tant du point de vue de leur tenue mécanique que de leur stabilité dans le temps ou d'apporter une nouvelle solution qui permet plus de versatilité dans la nature et la quantité d'actifs pouvant être incorporés au pansement ou patch.

Pour la fabrication, l'utilisation des charges minérales permet à la fois de jouer un rôle tensioactif lors de l'opération de film casting, de réduire le temps de séchage du film et de rigidifier le film.

Lors de l'utilisation du pansement patch, et en fonction du polymère utilisé, la présence d'un taux élevé de charge permet de réduire la sensibilité à l'hygrométrie du film et d'accélérer le temps de séchage sur la peau.

### EXPOSÉ DE L'INVENTION

L'invention concerne un pansement ou patch comprenant un film soluble à l'eau lequel comprend un polyoside d'origine naturelle et une charge minérale, caractérisé en ce que le film soluble comprend de 20 à 90 % en poids de charge minérale par rapport au poids total de matière sèche de la composition du film et en ce que la teneur en charge minérale est supérieure à celle en polyoside et en ce que la charge minérale représente une granulométrie moyenne de 1 à 15 micromètres.

Le polyoside d'origine naturelle (également appelé « polyoside naturel ») est avantageusement choisi parmi les polysaccharides et en particulier les extraits de plantes, d'algues ou d'animaux, en particulier d'animaux marins comme les dérivés de cellulose, les dérivés d'amidon, les dérivés de la chitine, les phycocolloides (tels que les alginates, les carraghénanes, les agars), les protéines végétales, les pectines, la gellane, le pullulan, la gomme arabique, en particulier les carraghénanes.

La charge minérale est avantageusement choisie parmi les phyllosilicates, comme le kaolin, le talc, la montmorillonite sodique ou calcique, en particulier le kaolin, ou d'autres formes minérales comme le mica, l'illite, la perlite, la diatomée ou les carbonates calcium.

L'invention concerne également un pansement ou patch comprenant un film soluble comprenant un polyoside et une charge minérale, comme support de composés actifs pour leur libération là où le pansement ou patch sera appliqué.

Les actifs sont de type hydrosolubles ou liposolubles, avantageusement choisis parmi les huiles essentielles, les phénols, les peptides, les protéines, les acides aminés, les vitamines, les glucides, les extraits aromatiques d'épices ou de plantes, etc.

La teneur en actifs dans le film soluble selon l'invention est avantageusement comprise entre 1 et 80 %.

Le pansement ou le patch selon l'invention a une forme géométrique déterminée, parallélépipédique, ovale, ovoide, etc. Il peut prendre la forme d'une bande parallélépipédique qui sera découpée au moment de son utilisation. Le pansement ou patch selon l'invention peut être constitué seulement du film soluble selon l'invention, ou bien comprendre l'association du film soluble avec un support, comme une couche imperméable associée à l'opposé de la face du film qui est destinée à adhérer à la peau ou la muqueuse.

Il se distingue en particulier d'un simple film par le fait qu'il est distribué dans un conditionnement approprié pour son usage en cosmétique ou en thérapie.

L'invention concerne également l'utilisation du pansement ou patch selon l'invention en cosmétique, et le pansement ou patch selon l'invention pour son utilisation en thérapie.

### DESCRIPTION DÉTAILLÉE DE L'INVENTION

L'invention concerne un pansement ou patch comprenant un film soluble à l'eau lequel comprend un polyoside d'origine naturelle et une charge minérale, caractérisé en ce que le film soluble comprend de 20 à 90 % en poids de charge minérale par rapport au poids total de matière sèche de la composition du film et en ce que la teneur en charge minérale est supérieure à celle en polyoside et en ce que la charge minérale représente une granulométrie moyenne de 1 à 15 micromètres.

Sauf indication contraire, les pourcentages sont donnés en poids par rapport au poids total de la matière sèche de la composition du film. Le poids de matière sèche peut être obtenu en faisant sécher le film jusqu'à évaporation complète de l'eau résiduelle dans des conditions qui ne viennent pas dégrader les constituants du film. Elle peut également être calculée en additionnant les poids de l'ensemble des constituants du film, à l'exception de l'eau.

Par film on entend une feuille d'épaisseur déterminée allant de 20 à 750 µm de préférence de 30 à 200 µm plus préférentiellement de 60 à 120 µm, par exemple de 60 à 90 µm.

Le polyoside d'origine naturelle est avantageusement choisi parmi les polysaccharides et en particulier les extraits de plantes, d'algues ou d'animaux, en particulier d'animaux marins comme les dérivés de cellulose (tels que la carboxymethyl cellulose, l'éthyl cellulose, le méthyl cellulose, l'éthylméthyl cellulose, l'éthyl-hydroxyéthyl cellulose, l'hydroxypropylmethyl cellulose, la cellulose microcristalline, la gomme de cellulose), les amidons et leurs dérivés (amidons modifiés, dextrines, carboxymethyl amidon), les dérivés de la chitine (chitosan), les phycocolloides (tels que les alginates, les carraghénanes, les agars), les pectines, le pullulan, les gommes (gomme arabique, gellane, de xanthane, de caroube, de guar, tara, karaya, ghatti, adragante, cassia), les cires (d'abeilles, candelilla, carnauba, de canne à sucre) en particulier les carraghénanes.

Compte tenu de leur haut poids moléculaire (par ex 150.000 à 500.00 Da) et de la longueur des chaines de monomères, les polymères d'origine naturelleprécités ont de bonnes propriétés filmogènes naturelles. Les propriétés filmogènes dépendent également de la présence de fonction hydrogènes (notamment de type OH ou COOH) qui favorisent les interactions entre les molécules et donc la formation de films. Parmi les polysaccharides, plusieurs familles de polymères répondent à cette définition et seront privilégiés pour former des films comme les dérivés de cellullose, les dérivés d'amidon, les dérivés de chitine (chitosane), les pectines ou les dérivés des algues (alginates, carraghénanes).

La charge minérale est avantageusement choisie parmi les phyllosilicates, comme le kaolin, le talc, la montmorillonite sodique ou calcique, l'attapulgite, en particulier le kaolin, ou d'autres formes minérales comme le mica, l'illite, la perlite, la vermicullite, l'halloysite, la diatomée, les carbonates ou bicarbonates (de calcium, de sodium, de potassium, de magnésium, d'ammonium), le marbre.

Selon l'invention, les charges minérales utilisées dans les pansements ou patchs présentent une granulométrie moyenne allant généralement de 1 à 15 µm. Un éventuel prétraitement par émulseur de type rotor-stator, par micro-onde, par microbroyage (type High Energy Ball Milling), par ultrason ou suspension dans l'eau, permet d'obtenir une exfoliation de la charge minérale et d'abaisser la taille des agrégats.

Selon un mode particulier de réalisation de l'invention, le film comprend également un agent tensioactif usuellement employé dans des compositions cosmétiques ou pharmaceutiques, notamment choisi parmi les agents tensioactifs non ioniques, anioniques ou cationiques, de préférence parmi les agents tensioactifs non ioniques. On citera en particulier les lécithines, les esters de sorbitan et d'acide gras, et leurs dérivés, notamment ceux commercialisés sous les dénominations Span et Tween (polysorbates), en particulier le monolaurate de sorbitan (Span 20), ou le sorbitan monostéarate. L'homme du métier saura identifier les agents tensioactifs adaptés selon la destination du produit selon l'invention.

Selon un autre mode particulier de réalisation de l'invention, le film comprend un agent plastifiant usuellement employé dans des compositions cosmétiques ou thérapeutiques. On citera en particulier le glycérol, le polyéthylène glycol et le sorbitol.

L'invention concerne également un film soluble comprenant un polyoside et une charge minérale, comme support de composés actifs pour leur libération là où le film sera appliqué sous forme de pansement ou de patch. En fonction de sa formulation, le film pourra jouer un rôle de support actif en influençant de manière bénéfique la libération des actifs sur et dans la peau, de manière accélérée ou retardée, ou en facilitant la pénétration transcutanée des actifs.

Par actif on entend selon l'invention tout composé minéral ou organique qui exerce une action biologique sur ou à travers la peau de l'individu sur lequel on l'applique. Cette activité biologique peut être une action antiseptique, antifongique ou antibiotique, une action hydratante, une action raffermissante, une action occlusive, une action désincrustante, une action destinée à favoriser un réchauffement musculaire local, une action antalgique locale, une action hémostatique, une action répulsive et/ou insecticide, ou toute autre action dermatologique.

Les actifs sont hydrosolubles ou liposolubles, avantageusement choisis parmi les huiles essentielles, les huiles végétales, les phénols, les peptides, les extraits d'épices ou de plantes, les protéines végétales et acides aminés, les vitamines, les glucides, l'urée, les acides (acide hyaluronique), leurs mélanges, leurs dérivés ou tout autre ingrédient ou principe actif d'intérêt pour des applications dermo-cosmétique ou de thérapeutique etc.

Parmi les actifs naturels, on peut citer les huiles essentielles reconnues pour leurs activités naturelles antiseptiques, antifongiques, antibactériennes ou cicatrisantes, les polyphénols reconnus pour leur activité antioxydante.

Il est entendu que les composés employés pour leurs propriétés plastifiantes, comme le la glycérine, ne font pas partie des actifs au sens de la présente invention.

Le film selon l'invention pourra comprendre tout autre additif usuellement employé dans des formulations cosmétiques ou pharmaceutiques, comme des colorants, des arômes, des parfums etc.

La teneur des constituants du film selon l'invention dépendra et sera adaptée par l'homme du métier selon la nature des constituants employés et les propriétés physicochimiques recherchées pour le film, comme ses propriétés physiques de résistance au stockage et au transport, sa capacité d'adhésion à la peau selon son degré d'humidification, sa vitesse de délitement dans l'eau, ou encore sa capacité à contrôler la libération des actifs incorporés et/ou en améliorant la pénétration transcutanée.

En particulier, on cherchera pour la réalisation d'un pansement ou d'un patch selon l'invention, à ce que la plasticité du film permette un usage sous forme de film souple. Le matériau ne doit pas être cassant et peut supporter une déformation suffisante pour une application sous forme de patch. Ces propriétés se caractérisent notamment par une mesure du Module de Young dont les valeurs seront généralement comprises en 500 et 2000 MPa (à 25°C - vitesse d'étirage 2 mm/min), et une Contrainte à la Rupture généralement comprise entre 15 et 30 MPa.

La mesure du Module de Young et de la Contrainte à la Rupture se fait selon les méthodes usuelles bien connues de l'homme du métier. On citera en particulier la méthode de traction / étirage uniaxial, en utilisant des éprouvettes de type haltère de 22 mm de longueur utile, 6 mm de largeur et d'environ 200 µm d'épaisseur sur une machine de traction de type Instron® 4466.

La teneur en charge minérale est comprise de 20 et 90% en poids, avantageusement d'au moins 25 %, en particulier entre 30 et 80 %, d'au moins 50% pour un mode avantageux de réalisation de l'invention. La teneur en charge dépendra de la présence ou l'absence d'actif et en particulier de la nature hydrophile ou lipophile de l'actif.

En présence d'actifs, la teneur en charge pourra être inférieure à 50% selon la quantité d'actifs comprise dans la composition finale.

La teneur en polyoside est avantageusement choisie de 5 à 49 % en poids, de préférence de 5 à 35 % en poids, avantageusement de 5 à 25 % en poids, avantageusement de 10 à 20 % en poids.

Selon un mode particulier de réalisation de l'invention, le rapport pondéral charge minérale / polyoside naturel est supérieur à 1, préférentiellement d'au moins 1,5, en particulier d'au moins 1,8, plus préférentiellement d'au moins 2, selon les usages et propriétés recherchées pour le pansement ou patch selon l'invention. Selon les propriétés du film recherchées par l'homme du métier, ce rapport pondéral peut aller jusqu'à 6. Pour certains modes de réalisation, ce rapport pondéral est compris entre 1,8 et 2,5, avantageusement d'environ 2. Pour d'autres modes de réalisation ce rapport pondéral est compris entre 3,5 et 4,5, d'environ 4.

La teneur en actifs dans le film soluble selon l'invention est avantageusement comprise entre 1 et 80 %, en particulier entre 1 et 50%.

La teneur en agent tensioactif lorsque présent dans la composition est avantageusement de 0.2% à 5%.

La teneur en agent plastifiant, lorsque présent dans la composition, peut aller jusqu'à à 40%. Plus généralement elle sera comprise entre 5 et 30 %, avantageusement de 10 % à 20 %. L'homme du métier saura choisir la teneur en plastifiant en fonction des propriétés physicochimiques du film du patch ou pansement selon l'invention.

De manière avantageuse, le film comprend un plastifiant, le rapport pondéral plastifiant / total des charges et des polyosides allant de 0,2 à 0,3. De manière préférée, ce rapport pondérale plastifiant / (charge minérale + polyoside) est d'environ 0,25.L'invention concerne en particulier les compositions préférées suivantes selon l'invention, avec ou sans actifs.

### Composition sans actif

| | |
|---|---|
| Charge minérale | 50 à 70% |
| Polymère naturel | 10 à 30% |
| Tensioactif | 0 à 2 % |
| Plastifiant | 0 à 30 % |

### Composition avec actif hydrosoluble

| | |
|---|---|
| Charge minérale | 40% à 60% |
| Polymère naturel | 10 à 25% |
| Tensioactif | 0 à 2 % |
| Plastifiant | 0 à 30 % |
| Actif hydrosoluble | 1 à 25% |

### Composition avec actif liposoluble

| | |
|---|---|
| Charge minérale | 25 à 50% |
| Polymère naturel | 5 à 25% |
| Tensioactif | 0 à 2 % |
| Plastifiant | 0 à 30 % |
| Actif liposoluble | 1 à 45% |

le rapport pondéral charge minéral / polyosides étant supérieur à 1, tel que défini ci-dessus, et
le rapport pondéral plastifiant / (charge minérale + polyoside) allant de 0,2 à 0,3, tel que défini ci-dessus.

Le temps de fragmentation des films selon l'invention dans l'eau froide est avantageusement de 10" à 1' ; la solubilisation complète est de 30" à plusieurs minutes.

Les films selon l'invention conservent une bonne tenue mécanique, nécessaire notamment à sa manipulation et une grande souplesse pour son utilisation comme pansement ou patch. Ils ne sont pas collants ni huileux et se présentent comme une forme galénique sèche.

L'invention concerne aussi un procédé de fabrication d'un film selon l'invention. L'homme du métier connaît différents procédés de fabrication de tels films. De manière préférentielle, le film est préparé par la technique dite de « film casting » dont le principe consiste à préparer le mélange sous forme liquide, de le déposer sur un support non collant, et d'évaporer l'eau en passant dans un système de séchage, incluant éventuellement infra-rouge, four et ventilation. L'homme du métier saura adapter les conditions opératoires de fabrication à la composition de la solution employée et au dispositif de séchage dont il dispose.

En particulier, et en fonction de la formulation, les paramètres de séchage seront optimisés pour permettre la formation d'un film homogène, sans bulles ni trous. Le temps de séchage peut aller jusqu'à 20' ou plus, préférentiellement de 3' à 8', la température va de 50 à 140°C, préférentiellement de 70 à 110°C, par exemple de 80°C.

Dans le four, la ventilation peut être réglée jusqu'à 2500 tpm, préférentiellement de 1000 à 1800 tpm et lorsque l'on utilise des infra-rouges on choisira une intensité allant préférentiellement jusqu'à 25%.

Après séchage, le film soluble selon l'invention a une humidité résiduelle pouvant aller jusqu'à environ 10%.

Préalablement à la formation et au séchage du film, un travail d'homogénéisation de la solution filmogène sera effectué. En particulier, dans le cas d'actifs ou de constituants lipidiques, une opération de microémulsion sera réalisée à l'aide d'un émulseur ou d'un mélangeur de type rotor-stator.

L'invention concerne un pansement ou patch comprenant un film soluble selon l'invention ou obtenu par le procédé selon l'invention. Le pansement ou le patch selon l'invention a une forme géométrique déterminée, parallélépipédique, ovale, ovoide, etc. La forme du pansement ou patch dépendra notamment de la manière et de la zone sur laquelle on cherche à l'appliquer. Il peut prendre la forme d'une bande parallélépipédique qui sera découpée au moment de son utilisation. Il peut avoir une forme parallélépipédique prédécoupée pour une application sur des zones planes ou tubulaires du corps humain ou animal (par exemple membres, abdomen, dos) Il peut prendre une forme ovoïde ou de haricot pour être appliquer sur des zones mobile du corps humain (par exemple articulations, dessous de paupières, etc.). Il peut prendre des formes plus complexes comme la forme d'un visage avec des prédécoupes pour les yeux et la bouche pour une application sur le visage.

Le pansement ou patch selon l'invention peut être constitué seulement du film soluble selon l'invention, ou bien comprendre l'association du film soluble avec un support, comme une couche imperméable ou antiadhésive associée à l'opposé de la face du film qui est destinée à adhérer à la peau ou la muqueuse, pour faciliter l'application ou la manipulation du film..

Il se distingue en particulier d'un simple film par le fait qu'il est distribué dans un conditionnement approprié pour son usage en cosmétique ou en thérapie.

De tels conditionnements comprennent notamment la mise en sachets individuels en plastique, aluminium ou matériaux complexes, sous forme de distributeur / dispenser ou en coffret.

L'invention concerne également l'utilisation du film selon l'invention en cosmétique, et le film selon l'invention pour son utilisation en thérapie.

Sans actif, il peut être utilisé pour ses propriétés apaisantes, occlusives et tenseurs, seul ou en combinaison avec des solutions ou des crèmes. Il permet alors de jouer un rôle de protection physique et de film barrière.

Avec actif, il peut être utilisé pour prolonger le temps de contact entre la peau et l'actif et améliorer son efficacité, par exemple en facilitant la pénétration transcutanée.

Les pansements ou patchs sont employés aisément. Pour une application sur une zone de peau sèche, il suffit d'humidifier légèrement la peau avant d'appliquer la face du film devant entrer en contact avec elle. Pour une application sur une zone de peau humide, par exemple avec une plaie, il suffit d'appliquer la face du film devant entrer en contact avec elle, à laquelle elle adhérera sans autre opération.

Après usage, le pansement ou patch est retiré par pelage, ou peut être éliminé par simple nettoyage (rinçage) à l'eau dans laquelle le film est soluble.

Les films tels que définis ci-dessus, avant leur mise en forme de patch ou de pansement et le cas échéant avant leur conditionnement, font également partie de la présente invention.

### EXEMPLES

### Exemple 1 Films

Les films de compositions suivantes sont préparés suivant le procédé décrit ci-dessus. Les rapports pondéraux C/P (charge minérale / polyosides) et PI/(C+P) (plastifiant / somme des charges minérales et polyosides) sont calculés. Leurs Module de Young, leur Contrainte à la Rupture et leur Déformation sont mesurés selon la méthode de traction / étirage uniaxial, en utilisant des éprouvettes de type haltère de 22 mm de longueur utile, 6 mm de largeur et d'environ 200 µm d'épaisseur sur une machine de traction de type Instron® 4466.

### Film 1 : Patch cosmétique sans actif

| | % dans le produit séché | % de matière sèche |
|---|---|---|
| Kaolin | 57,6 | 63,3 |
| Carraghénane | 14,4 | 15,8 |
| Glycérol | 18,0 | 19,8 |
| Span 20 | 1,0 | 1,1 |
| Eau | 9,1 | - |
| C/P | | 4,01 |
| PI/(C+P) | | 0,25 |
| Module de Young *E* | En MPa | 200 < *E* < 500 |
| Rupture σ | En MPa | 5 < σ < 30 |
| Déformation ε | En % | 5% < ε < 10% |

### Film 2 : Patch Cosmétique avec actif hydrosoluble

| | % dans le produit séché | % de matière sèche |
|---|---|---|
| Kaolin | 43,7 | 48,1 |
| Carraghénane | 10,9 | 12,0 |
| Glycérol | 13,7 | 15,0 |
| Span 20 | 0,7 | 0,8 |
| Actif hydrosoluble | 21,9 | 24,0 |
| Eau | 9,1 | - |
| C/P | | 4,01 |
| PI/(C+P) | | 0,25 |
| Module de Young *E* | En MPa | 200 < *E* < 500 |
| Rupture σ | En MPa | 5 < σ < 30 |
| Déformation ε | En % | 5% < ε < 10% |

### Film 3 : Patch Cosmétique avec actif liposoluble

| | % dans le produit séché | % de matière sèche |
|---|---|---|
| Kaolin | 32,1 | 35,3 |
| Carraghénane | 8,0 | 8,8 |
| Glycérol | 10,0 | 11,0 |
| Span 20 | 0,5 | 0,6 |
| Actif liposoluble | 40,2 | 44,2 |
| Eau | 9,1 | - |
| C/P | | 4,01 |
| PI/(C+P) | | 0,25 |
| Module d'Young *E* | En MPa | 200 < *E* < 500 |
| Rupture σ | En MPa | 5 < σ < 30 |
| Déformation ε | En % | 5% < ε < 10% |

### Film 4 : Pansement sans actif

| | % dans le produit séché | % de matière sèche |
|---|---|---|
| Kaolin | 47,8 | 52,6 |
| Carraghénane | 12,0 | 13,2 |
| HPMC-K | 12,0 | 13,2 |
| Glycérol | 17,9 | 19,7 |
| Span 20 | 1,2 | 1,3 |
| Eau | 9,1 | - |
| C/P | | 2,01 |
| PI/(C+P) | | 0,25 |
| Module de Young *E* | En MPa | 200 < *E* < 500 |
| Rupture σ | En MPa | 5 < σ < 30 |
| Déformation ε | En % | 5% < ε < 10% |

### Film 5 : Pansement avec actif hydrosoluble

| | % dans le produit séché | % de matière sèche |
|---|---|---|
| Kaolin | 41,1 | 45,2 |
| Carraghénane | 10,3 | 11,3 |
| HPMC-K | 10,3 | 11,3 |
| Glycérol | 15,4 | 16,9 |
| Span 20 | 1,0 | 1,1 |
| Actif hydrosoluble | 12,8 | 14,1 |
| Eau | 9,1 | - |
| C/P | | 2,00 |
| PI/(C+P) | | 0,25 |
| Module de Young *E* | En MPa | 200 < *E* < 500 |
| Rupture σ | En MPa | 5 < σ < 30 |
| Déformation ε | En % | 5% < ε < 10% |

### Film 6 : Pansement avec actif liposoluble

| | % dans le produit séché | % de matière sèche |
|---|---|---|
| Kaolin | 36,0 | 39,6 |
| Carraghénane | 9,0 | 9,9 |
| HPMC-K | 9,0 | 9,9 |
| Glycérol | 13,5 | 14,9 |
| Span 20 | 0,9 | 1,0 |
| Actif liposoluble | 22,5 | 24,8 |
| Eau | 9,1 | - |
| C/P | | 4,01 |
| PI/(C+P) | | 0,27 |
| Module de Young *E* | En MPa | 200 < *E* < 500 |
| Rupture σ | En MPa | 5 < σ < 30 |
| Déformation ε | En % | 5% < ε < 10% |

### Exemple 2 : Essais de fragmentation et de solubilisation

Les temps de fragmentation et de solubilisation dans l'eau sont mesurés selon une méthode consistant à mettre un morceau de film (taille 3 cm x 3 cm) dans un volume d'eau (100 ml) à température ambiante (18 à 25 °C) et sous agitation dans un erlen avec agitateur magnétique de laboratoire, de type IKA RH basic 2, en position médiane de vitesse, correspondant à une vitesse de rotation du barreau magnétique d'environ 500 à 800 tours par minute. Le temps de fragmentation correspond à la période de temps après immersion à partir de laquelle le film se casse en morceaux de taille variable ; le temps de solubilisation correspond à la période de temps après immersion à partir de laquelle le film est complètement dissous dans l'eau (plus aucun morceau n'est visible).

Les résultats sont rapportés dans le Tableau ci-dessous

| | **Film 1** | **Film 2** | **Film 3** | **Film 4** | **Film 5** | **Film 6** |
|---|---|---|---|---|---|---|
| **Fragmentation** | 11' | 11' | 12' | 8' | 8' | 10' |
| **Solubilisation** | 40' | 40' | 50' | 60' | 60' | 70' |

### Exemple 3 : Améliorations techniques

La capacité d'incorporer des charges minérales selon l'invention apporte à l'homme du métier de nouvelles solutions techniques pour la réalisation de tels films et leur usage comme pansement ou patch, tant sur leurs propriétés physicochimiques, que pour leur capacité à mieux incorporer des actifs lipophiles ou que dans leur procédé de préparation.
**3.1 Modification des propriétés mécaniques** : modification de la rigidité du matériau. En fonction de la nature du polymère et de la charge minérale, il est possible de modifier les mécaniques caractéristiques du film en fonction des propriétés recherchées par l'homme du métier.
   Par exemple, il est possible de modifier le module de Young d'un film composé de CMC + 20% glycerol de 555 MPa à plus de 800 MPa avec l'ajout de 4g de talc pour 1 g de polymère (soit un taux d'incorporation de 400%). A l'inverse, l'ajout de 400 % de kaolin dans un film à base d'alginate +20% de glycerol, permet de réduire le Module d'Young de 1100 MPa à 660 MPa.
**3.2 Abaissement de la sensibilité à l'hygrométrie** : les films réalisés à partir de dérivés de celluloses ou d'hydrocolloides sont généralement très sensibles à l'hygrométrie. Il a été démontré que l'incorporation d'une quantité importante de charge minérale (ratio minéral / polymère > 2) permettait d'abaisser cette sensibilité à l'hygrométrie du film lors d'un conservation à des conditions de 25°C - 75% HR. Le film avec charge minéral est beaucoup moins collant qu'un film sans charge ; il est également possible de superposer des couches de films sans adhésion entre elles, ce qui n'est pas possible pour un film sans charge ou faiblement chargé.
**3.3 Faciliter l'incorporation d'actifs lipidiques/lipophiles** : l'incorporation d'un lipide dans une solution polymère sur base aqueuse nécessite une préparation particulière, et se traduit généralement par un relargage du lipide lors de la conservation du film (effet gras). Il a été montré que l'incorporation d'une quantité importante de certains minéraux, en particulier le kaolin, (ratio minéral / polymère > 2) facilitait le travail d'émulsion et l'introduction d'un actif lipidique à l'intérieur d'un film. De plus, l'affinité de la charge minérale avec les lipides permet de limiter le phénomène de relargage, autorisant la réalisation de formules contenant plus de lipides, sans donner un effet gras au film.
**3.4 Rôle tensioactif :** lors de la fabrication d'un film par une méthode de film casting, il a été montré que l'incorporation d'une quantité importante de charge minérale (ratio minéral / polymère > 2) joue un rôle tensioactif bénéfique pour limiter l'adhésion sur le support de casting (ex support téflon) et garantir l'homogénéité du film.
**3.5 Amélioration des conditions de séchage** lors de la fabrication par la technique de film casting : il a été montré qu'il était possible
   1) d'abaisser la température de séchage de 90-95 °C à 70-75°C (pour un temps de séchage de 7 min) par l'incorporation de 200% de kaolin dans un film de carraghénane à 1%, ou
   2) d'abaisser le temps de séchage de 7 min à 5 min pour ce même film à une température constante de 90°C.

### Exemple 4 : utilisation comme pansement vétérinaire

Le film peut être utilisé comme pansement ou patch à usage vétérinaire. La zone à protéger est préalablement nettoyée et humidifiée, le film est appliqué sur la peau humide de l'animal. Si besoin, le pansement pourra également être humidifié par pulvérisation ou brumisation d'eau. En s'humidifiant le film adhère à la peau ou au poil, puis sèche en environ 5-10 min. Une fois séché, le pansement colle de façon durable à la peau ou au poil de l'animal.

En fonction des conditions, le pansement pourra au choix : soit être rincé à l'eau, en appliquant de l'eau froide ou tiède et en frottant légèrement ; dans ce cas le pansement se solubilisera et disparaîtra en quelques dizaines de secondes, soit être laissé sur la peau ; dans ce cas au bout de quelques temps le pansement commencera à se fragmenter, puis progressivement sous l'action conjuguée de la sudation et des frottements se délitera jusqu'à disparition complète.

### RÉFÉRENCES

- EP 7 50 905
- EP 613 692
- US 7,491,406
- US 2006/027873
- Sanchez Garcia & al, J. Agric. Food Chem. 2010, 58, 6884-6894
- Tunç & Duman, Applied Clay Science, 5010, 48, 414-424
- Wilhelm & aL., Carbohydrate Polymers, 2003, 52, 101-110
- Carvalho & al., Carbohydrate Polymers, 2001, 45, 189-194
- Savanan & al., Elixir Appl. Chem., 2012, 44, 7374-7377

## Revendications

1. Pansement ou patch comprenant un film soluble à l'eau lequel comprend un polyoside d'origine naturelle et une charge minérale, **caractérisé en ce que** le film soluble comprend de 20 à 90 % en poids de charge minérale par rapport au poids total de matière sèche de la composition du film et **en ce que** la teneur en charge minérale est supérieure à celle en polyoside et **en ce que** la charge minérale représente une granulométrie moyenne de 1 à 15 µm.

2. Pansement ou patch selon la revendication 1, **caractérisé en ce que** le polyoside d'origine naturelle est choisi parmi les polysaccharides.

3. Pansement ou patch selon la revendication 2, **caractérisé en ce que** le polysaccharide est choisi parmi les polysaccharides et en particulier les extraits de plantes, d'algues ou d'animaux, en particulier d'animaux marins.

4. Pansement ou patch selon la revendication 3, **caractérisé en ce que** le polysaccharide est choisi parmi les dérivés de cellulose, les amidons et leurs dérivés, les dérivés de la chitine, les phycocolloides, les pectines, le pullulan, les gommes, les cires.

5. Pansement ou patch selon la revendication 3, **caractérisé en ce que** le polysaccharide est choisi parmi les caraghénanes.

6. Pansement ou patch selon l'un des revendications 1 à 5, **caractérisé en ce qu'**il comprend de 5 à 49 % en poids de polyoside.

7. Pansement ou patch selon l'une des revendications 1 à 6, **caractérisé en ce que** la charge minérale est choisie parmi les phyllosilicates, comme le kaolin, le talc, la montmorillonite sodique ou calcique, l'attapulgite, en particulier le kaolin, ou d'autres formes minérales comme le mica, l'illite, la perlite, la vermicullite, l'halloysite, la diatomée, les carbonates ou bicarbonates (de calcium, de sodium, de potassium, de magnésium, d'ammonium), le marbre.

8. Pansement ou patch selon la revendication 7, **caractérisé en ce que** le phyllosilicate est du kaolin.

9. Pansement ou patch selon l'une des revendications 1 à 8, **caractérisé en ce qu'**il comprend de 30 à 80 % en poids de charge minérale.

10. Pansement ou patch selon l'une des revendications 1 à 7, **caractérisé en ce que** le film soluble comprend un agent tensioactif et/ou un agent plastifiant usuellement employés dans des compositions cosmétiques ou pharmaceutiques.

11. Pansement ou patch selon l'une des revendications 1 à 10, **caractérisé en ce que** le film soluble comprend au moins un actif hydrosoluble ou liposoluble choisi parmi les huiles essentielles, les phénols, les peptides, des extraits d'épices ou de plantes.

12. Pansement ou patch selon l'une des revendications 1 à 11, **caractérisé en ce que** le film soluble comprend de 1 et 50 % d'actif.

13. Pansement ou patch selon l'une des revendications 1 à 12, caractérisé que le rapport pondéral charge minérale / polyoside d'origine naturelle est d'au moins 1,5.

14. Pansement ou patch selon la revendication 13, **caractérisé en ce que** le rapport pondéral charge minérale / polyoside d'origine naturelle est compris entre 1,8 et 2,5.

15. Pansement ou patch selon la revendication 13, **caractérisé en ce que** le rapport pondéral charge minérale / polyoside d'origine naturelle al est compris entre 3,5 et 4,5.

16. Pansement ou patch selon l'une des revendications 1 à 13, **caractérisé en ce que** le film comprend un plastifiant et le rapport pondéral plastifiant / total des charges et des polyosides va de 0,2 à 0,3.

17. Pansement ou patch selon l'une des revendications 1 à 16, **caractérisé en ce que** le film soluble est choisi parmi les compositions suivantes :
**Composition sans actif**
| | |
|---|---|
| Charge minérale | 50 à 70% |
| Polymère naturel | 10 à 30% |
| Tensioactif | 0 à 2 % |
| Plastifiant | 0 à 30 % |
**Composition avec actif hydrosoluble**
| | |
|---|---|
| Charge minérale | 40% à 60% |
| Polymère naturel | 10 à 25% |
| Tensioactif | 0 à 2 % |
| Plastifiant | 0 à 30 % |
| Actif hydrosoluble | 1 à 25% |
**Composition avec actif liposoluble**
| | |
|---|---|
| Charge minérale | 25 à 50% |
| Polymère naturel | 5 à 25% |
| Tensioactif | 0 à 2 % |
| Plastifiant | 0 à 30 % |
| Actif liposoluble | 1 à 45% |

18. Pansement ou patch selon l'une des revendications 1 à 17, **caractérisé en ce que** l'épaisseur du film soluble va de 20 à 750 µm

19. Procédé de fabrication d'un film tel que défini dans l'une des revendications l'une des revendications 1 à 18, **caractérisé en ce qu'**il comprend les étapes de
- préparer un mélange des constituants du film sous forme liquide aqueuse,
- de déposer le mélange précédemment obtenu sur un support approprié, et
- d'évaporer l'eau en passant dans un système de séchage.

20. Film soluble tel que défini dans l'une des revendications 1 à 17, **caractérisé en ce que** son épaisseur va de 20 à 750 µm.

21. Utilisation cosmétique d'un pansement ou patch selon l'une des revendications 1 à 18.

## Patentansprüche

1. Verband oder Pflaster mit einem wasserlöslichen Film, der eine Polyose natürlichen Ursprungs und einen mineralischen Füllstoff umfasst, **dadurch gekennzeichnet, dass** der lösliche Film 20 bis 90 Gew.-% an mineralischem Füllstoff bezogen auf das Gesamtgewicht der Trockenmasse der Zusammensetzung des Films aufweist, dass der Gehalt an mineralischem Füllstoff größer ist als der der Polyose ist und dass der mineralische Füllstoff eine durchschnittliche Partikelgröße von 1 bis 15 µm aufweist.

2. Verband oder Pflaster nach Anspruch 1, **dadurch gekennzeichnet, dass** die Polyose natürlichen Ursprungs aus den Polysacchariden ausgewählt ist.

3. Verband oder Pflaster nach Anspruch 2, **dadurch gekennzeichnet, dass** das Polysaccharid aus den Polysacchariden und insbesondere aus den Extrakten von Pflanzen, Algen oder Tiere, insbesondere Meerestiere ausgewählt ist.

4. Verband oder Pflaster nach Anspruch 3, **dadurch gekennzeichnet, dass** das Polysaccharid aus Cellulosederivaten, Stärken und ihren Derivaten, Derivaten von Chitin, Phycokolloiden, Pektinen, Pullulan, Gummis und Wachsen ausgewählt ist.

5. Verband oder Pflaster nach Anspruch 3, **dadurch gekennzeichnet, dass** das Polysaccharid aus den Carrageenanen ausgewählt ist.

6. Verband oder Pflaster nach einem der Ansprüche 1 bis 5, **dadurch gekennzeichnet, dass** dieser oder dieses 5 bis 49 Gew.-% Polyose umfasst.

7. Verband oder Pflaster nach einem der Ansprüche 1 bis 6, **dadurch gekennzeichnet, dass** der mineralische Füllstoff aus Schichtsilikaten wie Kaolin, Talkum, Natrium- oder Calciummontmorillonit, Attapulgit, insbesondere Kaolin, oder aus andere mineralischen Formen wie Glimmer, Illit, Perlit, Vermiculit, Halloysit, Kieselgur, Carbonate oder Bicarbonate (von Calcium, Natrium, Kalium, Magnesium oder Ammonium) oder Marmor ausgewählt ist.

8. Verband oder Pflaster nach Anspruch 7, **dadurch gekennzeichnet, dass** das Schichtsilikat Kaolin ist.

9. Verband oder Pflaster nach einem der Ansprüche 1 bis 8, **dadurch gekennzeichnet** dieser oder dieses 30 bis 80 Gew.-% mineralischen Füllstoff umfasst.

10. Verband oder Pflaster nach einem der Ansprüche 1 bis 7, **dadurch gekennzeichnet, dass** der lösliche Film ein Tensid und/oder einen Weichmacher aufweist, die üblicherweise in kosmetischen oder pharmazeutischen Zusammensetzungen verwendet werden.

11. Verband oder Pflaster nach einem der Ansprüche 1 bis 10, **dadurch gekennzeichnet, dass** der lösliche Film mindestens einen wasserlöslichen oder fettlöslichen Wirkstoff umfasst, der aus den ätherischen Ölen, den Phenolen, Peptiden oder aus den Extrakten von Gewürzen oder Pflanzen ausgewählt ist.

12. Verband oder Pflaster nach einem der Ansprüche 1 bis 11, **dadurch gekennzeichnet, dass** der lösliche Film zwischen 1 bis 50% Wirkstoff aufweist.

13. Verband oder Pflaster nach einem der Ansprüche 1 bis 12, **dadurch gekennzeichnet, dass** das Gewichtsverhältnis von mineralischem Füllstoff / Polyose natürlichen Ursprungs mindestens 1,5 beträgt.

14. Verband oder Pflaster nach Anspruch 13, **dadurch gekennzeichnet, dass** das Gewichtsverhältnis von mineralischem Füllstoff / Polyose natürlichen Ursprungs zwischen 1,8 und 2,5 liegt.

15. Verband oder Pflaster nach Anspruch 13, **dadurch gekennzeichnet, dass** das Gewichtsverhältnis von mineralischem Füllstoff / Polyose natürlichen Ursprungs zwischen 3,5 und 4,5 liegt.

16. Verband oder Pflaster nach einem der Ansprüche 1 bis 13, **dadurch gekennzeichnet, dass** der Film einen Weichmacher aufweist und dass das Verhältnis Weichmacher / Gesamtgewicht der Füllstoffe und Polyosen zwischen 0,2 und 0,3 liegt.

17. Verband oder Pflaster nach einem der Ansprüche 1 bis 16, **dadurch gekennzeichnet, dass** der lösliche Film aus folgenden Zusammensetzungen ausgewählt ist:
**Zusammensetzung ohne Wirkstoff**
| | |
|---|---|
| Mineralischer Füllstoff | 50 bis 70% |
| Natürliches Polymer | 10 bis 30% |
| Tensid | 0 bis 2% |
| Weichmacher | 0 bis 30% |
**Zusammensetzung mit wasserlöslichem Wirkstoff**
| | |
|---|---|
| Mineralischer Füllstoff | 40% bis 60% |
| Natürliches Polymer | 10 bis 25% |
| Tensid | 0 bis 2% |
| Weichmacher | 0 bis 30% |
| Wasserlöslicher Wirkstoff | 1 bis 25% |
**Zusammensetzung mit fettlöslichem Wirkstoff**
| | |
|---|---|
| Mineralischer Füllstoff | 25 bis 50% |
| Natürliches Polymer | 5 bis 25% |
| Tensid | 0 bis 2% |
| Weichmacher | 0 bis 30% |
| Fettlöslicher Wirkstoff | 1 bis 45% |

18. Verband oder Pflaster nach einem der Ansprüche 1 bis 17, **dadurch gekennzeichnet**, die Dicke des löslichen Films im Bereich von 20 bis 750 µm liegt

19. Verfahren zur Herstellung eines Films nach einem der Ansprüche 1 bis 18, **dadurch gekennzeichnet, dass** es die Schritte umfasst,
- eine Mischung der Bestandteile des Films in wässriger flüssiger Form herzustellen,
- die zuvor erhaltene Mischung auf einem geeigneten Träger abzulegen und
- das Wasser beim Durchlauf durch ein Trocknungssystem zu verdampfen.

20. Löslicher Film nach einem der Ansprüche 1 bis 17, **dadurch gekennzeichnet, dass** seine Dicke im Bereich von 20 bis 750 µm liegt.

21. Kosmetische Verwendung eines Verbands oder Pflasters gemäß einem der Ansprüche 1 bis 18.

## Claims

1. A plaster or patch comprising a water-soluble film that comprises a polyoside of natural origin and a mineral filler, **characterised in that** the soluble film comprises from 20% to 90% by weight mineral filler with respect to the total weight of dry matter of the composition of the film, and **in that** the mineral filler content is greater than the polyoside content and **in that** the mineral filler represents a mean particle size of 1 to 15 µm.

2. A plaster or patch according to claim 1, **characterised in that** the polyoside of natural origin is chosen from the polysaccharides.

3. A plaster or patch according to claim 2, **characterised in that** the polysaccharide is chosen from polysaccharides and in particular extracts of plants, algae or animals, in particular marine animals.

4. A plaster or patch according to claim 3, **characterised in that** the polysaccharide is chosen from cellulose derivatives, starches and derivatives thereof, chitin derivatives, phycocolloids, pectins, pullulan, gums and waxes.

5. A plaster or patch according to claim 3, **characterised in that** the polysaccharide is chosen from carrageenans.

6. A plaster or patch according to one of claims 1 to 5, **characterised in that** it comprises from 5% to 49% by weight polyoside.

7. A plaster or patch according to one of claims 1 to 6, **characterised in that** the mineral filler is chosen from phyllosilicates such as kaolin, talc, sodium or calcium montmorillonite, attapulgite, in particular kaolin, or other mineral forms such as mica, illite, perlite, vermiculite, halloysite, diatom, carbonates or bicarbonates (of calcium, sodium, potassium, magnesium or ammonium), or marble.

8. A plaster or patch according to claim 7, **characterised in that** the phyllosilicate is kaolin.

9. A plaster or patch according to one of claims 1 to 8, **characterised in that** it comprises from 30% to 80% by weight mineral filler.

10. A plaster or patch according to one of claims 1 to 7, **characterised in that** the soluble film comprises a surfactant and/or a plasticiser normally used in cosmetic or pharmaceutical compositions.

11. A plaster or patch according to one of claims 1 to 10, **characterised in that** the soluble film comprises at least one water-soluble or liposoluble active agent chosen from essential oils, phenols, peptides, or spice or plant extracts.

12. A plaster or patch according to one of claims 1 to 11, **characterised in that** the soluble film comprises from 1% to 50% active agent.

13. A plaster or patch according to one of claims 1 to 12, **characterised in that** the ratio by weight of mineral filler to polyoside of natural origin is at least 1.5.

14. A plaster or patch according to claim 13, **characterised in that** the ratio by weight of mineral filler to polyoside of natural origin is between 1.8 and 2.5.

15. A plaster or patch according to claim 13, **characterised in that** the ratio by weight of mineral filler to polyoside of natural origin is between 3.5 and 4.5.

16. A plaster or patch according to one of claims 1 to 13, **characterised in that** the film comprises a plasticiser and the ratio by weight of plasticiser to total fillers and polyosides ranges from 0.2 to 0.3.

17. A plaster or patch according to one of claims 1 to 16, **characterised in that** the soluble film is chosen from the following compositions:
**Composition without active agent**
| | |
|---|---|
| mineral filler | 50% to 70% |
| natural polymer | 10% to 30% |
| surfactant | 0% to 2% |
| plasticiser | 0% to 30% |
**Composition with water-soluble active agent**
| | |
|---|---|
| mineral filler | 40% to 60% |
| natural polymer | 10% to 25% |
| surfactant | 0% to 2% |
| plasticiser | 0% to 30% |
| water-soluble active agent | 1% to 25% |
**Composition with liposoluble active agent**
| | |
|---|---|
| mineral filler | 25% to 50% |
| natural polymer | 5% to 25% |
| surfactant | 0% to 2% |
| plasticiser | 0% to 30% |
| liposoluble active agent | 1% to 45% |

18. A plaster or patch according to one of claims 1 to 17, **characterised in that** the thickness of the soluble film ranges from 20 to 750 µm.

19. A method for manufacturing a film as defined in one of claims 1 to 18, **characterised in that** it comprises the steps of
- preparing a mixture of constituents of the film in aqueous liquid form,
- depositing the mixture previously obtained on a suitable substrate, and
- evaporating the water by passing through a drying system.

20. A soluble film as defined in one of claims 1 to 17, **characterised in that** the thickness thereof ranges from 20 to 750 µm.

21. Cosmetic use of a plaster or patch according to one of claims 1 to 18.
